# EUROPÄISCHE PATENTSCHRIFT

(11) **EP 1 455 934 B1**
(45) Veröffentlichungstag und Bekanntmachung des Hinweises auf die Patenterteilung: **19.11.2008**
(21) Anmeldenummer: 02792877.9
(22) Anmeldetag: 04.12.2002
(51) Int. Cl.: B01J 23/30, C07C 29/56, C07C 33/03

(54) **VERFAHREN ZUR ISOMERISIERUNG VON ALLYLALKOHOLEN**
METHOD FOR ISOMERIZING ALLYL ALCOHOLS
PROCEDE D'ISOMERISATION D'ALCOOLS ALLYLIQUES

(30) Priorität: 07.12.2001 DE 10160147
(43) Veröffentlichungstag der Anmeldung: 15.09.2004
(73) Patentinhaber: BASF SE, 67056 Ludwigshafen (DE)
(72) Erfinder: HAESE, Frank, 67061 Ludwigshafen (DE); EBEL, Klaus, 68623 Lampertheim (DE)
(86) Internationale Anmeldenummer: PCT/EP2002/013690
(87) Internationale Veröffentlichungsnummer: WO 2003/047749

(56) Entgegenhaltungen:
- WO-A-01/42177
- HOSOGAI T ET AL: "Selective allyic rearrangement with tungsten catalyst" CHEMISTRY LETTERS, CHEMICAL SOCIETY OF JAPAN. TOKYO, JP, Nr. 3, März 1982 (1982-03), Seiten 357-360, XP002165951 ISSN: 0366-7022 in der Anmeldung erwähnt
- HERRMANN, W. A. ET AL: "Chiral molybdenum(VI) and tungsten(VI) 2'- pyridinyl alcoholate complexes. Synthesis, structure and catalytic properties in asymmetric olefin epoxidation" JOURNAL OF ORGANOMETALLIC CHEMISTRY (2000), 603(1), 69-79 , XP004213440
- WONG, YEE-LOK ET AL: "Synthesis, electrochemistry, and oxygen-atom transfer reactions of dioxotungsten (VI) and -molybdenum(VI) complexes with N2O2 and N2S tetradentate ligands" EUROPEAN JOURNAL OF INORGANIC CHEMISTRY (1999), (2), 313-321 , XP002237417

## Beschreibung

Die vorliegende Erfindung betrifft ein Verfahren zur Isomerisierung von Edukt-Allylalkoholen zu Produkt-Allylalkoholen in beiden Richtungen des Gleichgewichtes, dadurch gekennzeichnet, dass die Umsetzung in Gegenwart eines Dioxowolfram(VI)-Komplexes stattfindet sowie neue Dioxowolfram(VI)-Komplexe und deren Verwendung.

Allylalkohole stellen wichtige Zwischenprodukte in der industriellen organischen Produktsynthese dar. Tertiäre Allylalkohole insbesondere dienen zum Beispiel als Zwischenverbindungen bei der Herstellung von Riechstoffen oder auch als Additive in Seifen und Detergentien.

Allylalkohole isomerisieren unter Säurekatalyse. Diese Isomerisierung entspricht einer 1,3-Wanderung der Hydroxylgruppe und einer internen Verschiebung der Doppelbindung, wie in der folgenden Gleichung mit den allgemeinen Formeln I und II dargestellt: 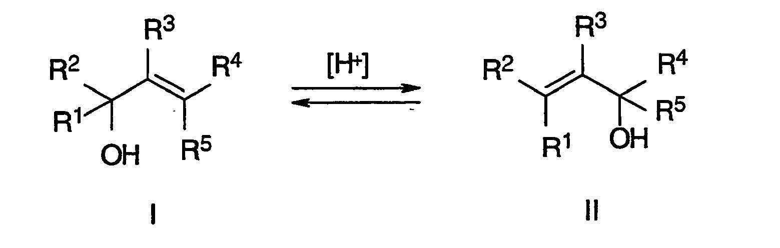 wobei die Reste R1 bis R5 Wasserstoff oder einen ein oder mehrfach ungesättigten oder gesättigten C₁-C₁₂-Alkylrest bedeuten, der gegebenenfalls substituiert sein kann.

Das Verfahren ist besonders geeignet für die Herstellung von tertiären Produkt-Allylalkoholen, wie z.B. 2-Linalool, durch Isomerisieren von primären oder sekundären Allylalkoholen, wie beispielsweise Geraniol oder Nerol.

Geraniol (2-trans-3,7-Dimethyl-2,6-octadien-8-ol), Nerol (2-cis-3,7-Dimethyl-2,6-octadien-8-ol) und 2-Linalool (3,7-Dimethyl-1,6-octadien-3-ol) sind wichtige Verbindungen in der Riechstoffindustrie. Sie werden sowohl direkt als Riechstoffe eingesetzt oder durch Umsetzung mit anderen Verbindungen zu höher molekularen Riechstoffen umgesetzt. Bedeutung besitzen diese Terpenalkohole auch als C₁₀-Bausteine in der Synthese von Vitaminen, wie dem Vitamin E und dem Vitamin A.

In der Literatur wurden in der Vergangenheit bevorzugt Verfahren zur Isomerisierung von Linalool zu Geraniol beschrieben. Da es sich bei den Isomerisierungen um Gleichgewichtsreaktionen handelt, sind die entwickelten Verfahren prinzipiell auch für die umgekehrte Reaktion der Isomerisierung von Geraniol oder Nerol zu Linalool anwendbar.

Zunächst wurden die Isomerisierungsreaktionen von Allylalkoholen mit Säuren als Katalysatoren durchgeführt. Diese Verfahren waren jedoch nur von eingeschränkter Bedeutung, da bei ihnen Nebenreaktionen, wie z.B. Dehydratisierungen oder Cyclisierungen dominierten.

Später wurden Molybdän-, Vanadium- und Wolfram-Verbindungen als Katalysatoren für die Umlagerung von substituierten Allylalkoholen identifiziert und untersucht (vgl. P. Chabardes et al. in Tetrahedron 33 (1977), Seiten 1775-1783.).

Während die in GB 125 6184 als Isomerisierungskatalysator beschriebene Molybdänverbindung unbefriedigende Reaktionsergebnisse ergab, waren mit Wolframoxo(VI)alkoholat-Katalysatoren der Formel WO(OR)₄ in Gegenwart einer Stickstoff-Base als zusätzlichem Liganden relativ hohe Selektivitäten bei gleichzeitig höheren Aktivitäten als mit den analogen Vanadiumoxo(V)alkoholat-Katalysatoren der Formel VO(OR)₃ möglich. Weitere Vorteile der Wolfram-Katalysatoren liegen darin, daß sie sich zum einen leicht aus dem Reaktionsgemisch abtrennen lassen (vgl. T. Hosogai et al. in Chemistry Letters 1982, Seiten 357-360) und daß sie im Vergleich zum Vanadiumkatalysator eine nur geringe Toxizität aufweisen.

Weiterhin ist aus DE 25 16 698 die Herstellung von neuen Katalysatoren auf der Basis von Wolfram, sowie deren Verwendung für die katalytische Umlagerung von tertiären zu primären Allylalkoholen bekannt. Als Katalysatoren werden bei diesem Verfahren Wolframoxo(VI)-Komplexe enthaltend Alkoxy-Reste und/oder über Sauerstoff gebundene Trialkylsilyl-Reste beschrieben, die zur Verbesserung der Selektivität zusätzlich koordinativ an das Wolfram gebundene Liganden enthalten, welche ein Element ausgewählt aus den Elementen N, P, As und Bi, insbesondere Liganden ausgewählt aus der Klasse der primären, sekundären und tertiären Monoamine, der Polyamine, der Schiff'schen Basen, der Imine, Nitrile und Isonitrile enthalten. Als besonders geeignete Liganden werden hierin genannt primäre Monoamine, wie Methylamin, Ethylamin, Propylamin, β-Ethoxy-ethylamin, Butylamin, Cyclohexylamin, Anilin und Naphthylamin; sekundäre Monoamine, wie Dimethylamin, Diethylamin, Dibutylamin, Dicyclohexylamin, Methylanilin, Methylcyclohexylamin, Piperidin, Morpholin und Pyrrolidin; tertiäre Monoamine, wie Trimethylamin, Triethylamin, Ethyldibutylamin, Tricyclohexylamin, Dimethylanilin, Pyridin, Pikolin, Chinolin, Isochinolin, N-Methylpyrrolidin und N-Methyl-morpholin; Ethylendiamin, Pyrazin, Piperazin, Pyrimidin, Triethylendiamin, 1,8-Diaza-bicyclo[5,4,0]undec-7-en, Polyethylenimine sowie Ionenaustauscherharze mit einer Vielzahl von Aminogruppen innerhalb der Moleküle, insbesondere Pyridin, Triethylamin, Cyclohexylamin, Diethylamin und Tricyclohexylphosphin. Aminoalkohole werden hierin nicht genannt.

Eigene Untersuchungen zur Isomerisierung von Geraniol mit einer 0,05 mol-%-igen Lösung von Wolframoxo(VI)-tetrakisgeranylat analog zum dem in DE 25 16 698 beschriebenen Verfahren zeigten, dass die Umlagerung zu Linalool in Gegenwart einer Stickstoff-Base bei 200°C (Reaktionszeit etwa 1 Stunde) deutlich selektiver verläuft als ohne Mitverwendung einer Stickstoff-Base. Als Stickstoffbasen wurden hierbei Diethylamin, Pyridin, Imidazol und Poly-(4-vinylpyridin) eingesetzt. Verbesserungen wurden durch den Zusatz von Aminoalkoholen zur Katalysatorlösung erreicht (siehe Vergleichsversuche, Beispiele 2 bis 6). Die mit diesem Verfahren erzielten Selektivitäten an Linalool sind recht gut, doch lassen die dabei erzielten Umsätze noch zu wünschen übrig.

Nachteilig wirkten sich bei diesen Versuchen also die vergleichsweise geringeren Umsätze bei gleichzeitig hohen Temperaturen von über 150°C aus, welche die Bildung von Nebenprodukten beschleunigten.

DE 25 16 698 beschreibt ebenfalls die Synthese von Wolframalkoholaten, durch Alkoholyse von Wolframoxotetrachlorid mit Alkoholen oder in Alkoholen gelösten Alkoholaten. Die Abtrennung des Chlorids in Form von Ammoniumchlorid mit Ammoniak oder als Natriumchlorid mit Natriummethylat ist zwar möglich aber stets unvollständig. In dem destillierten Produkt-Allylalkohol ist fast immer auch Chlor vorhanden, was dessen Qualität und Akzeptanz für Riechstoffe und Vitamine stark beeinträchtigt. Zusätzlich bewirkt das Chlorid im Wolframalkoholat selbst in ppm-Mengen nachteilig Korrosion in Metall-Kolonnen und -reaktoren. Eine nachträgliche Abtrennung von Chlorid-Spuren über Aktivkohle-Filter oder Silber(I)oxid ist möglich, aber aufwendig und macht das Gesamtverfahren komplex und teurer.

Zudem muss Wolframoxotetrachlorid nach bekannten Methoden mit Wolframsäure und Thionylchlorid in einer vorgelagerten Reaktion erst bereitgestellt werden, so dass die Synthese von Wolframalkoholaten mit hohem Aufwand in einem mehrstufigen Verfahren erfolgen muss.

Das ebenfalls in DE 25 16 698 beschriebene Verfahren für Wolframoxo(VI)-alkoholat-Komplexe, ausgehend von Wolframtrioxid mit Hydroxyverbindungen, ist aufgrund der schlechten Ausbeuten für großtechnische Anwendungen nicht brauchbar.

Wolframalkoholate sind bekanntermaßen hydrolyseempfindlich und reagieren mit Wasser zu Alkohol und Wolframoxid. Da als Nebenreaktion der Allylalkohol-Isomerisierung stets auch Dehydratisierung erfolgt, fallen unter den Bedingungen der Allylalkohol-Isomerisierung zunehmend Wolframoxide schwerlöslich aus der Reaktionsmischung aus. Diese erhöhen die Katalysatorkosten und begünstigen weiter die Bildung von Nebenprodukten sowie Wasser aus Allylalkoholen und führen zur weiteren Verschlechterung der Selektivität.

Das Patent WO 0142177 beschreibt ein Verfahren zur Isomerisierung von Allylalkoholen in Gegenwart von Wolframoxo (VI)-Komplexen enthaltend Aminoalkoholen Liganden.

Es war daher die Aufgabe der Erfindung, das Verfahren zur Isomerisierung von Allylalkoholen weiter so zu verbessern, dass der Wolframoxokatalysator Halogen-frei und in einfachen, kostengünstigen Schritten hergestellt werden kann, um eine Kontamination des Allylalkohols mit Halogen und darüber hinaus Korrosionsprobleme in der Anlage zu vermeiden. Weiterhin sollten durch Hydrolyse bedingte Katalysatorverluste verringert oder ganz vermieden und die Produktselektivität verbessert werden. Auch besteht weiterhin der Bedarf, die Geschwindigkeit der Gleichgewichtseinstellung zu beschleunigen und die Raumzeitausbeuten zu steigern, ohne dabei die Isomerisierungs-Temperatur weiter anheben zu müssen.

Weiterhin sollten die verbesserten Katalysatoren bei der Isomerisierung von primären oder sekundären Allylalkoholen, wie Geraniol und Nerol, zu tertiären Allylalkoholen, wie Linalool, höhere Umsätze des eingesetzten Edukt-Allylalkohols erzielen, d.h. die Geschwindigkeit der Gleichgewichtseinstellung beschleunigen. Außerdem sollten die neuen Katalysatoren einfacher herstellbar sein als die bisherigen.

Erfindungsgemäß wurde die Aufgabe mit neuen homogen gelösten Dioxowolfram(VI)-Komplexen der allgemeinen Formel (III) gelöst, bei gleichzeitig verbesserter Selektivität und höherer Aktivität für die Isomerisierung von Geraniol und Nerol zu Linalool.

Es können erfindungsgemäß sowohl homogene Lösungen von Dioxowolfram(VI) in Wasser, Alkohol oder einem anderen Lösungsmittel verwendet werden.

Gegenstand der Erfindung ist ein Verfahren zur Isomerisierung von Edukt-Allylalkoholen der allgemeinen Formel (I) zu Produkt-Allylalkoholen der allgemeinen Formel (II) 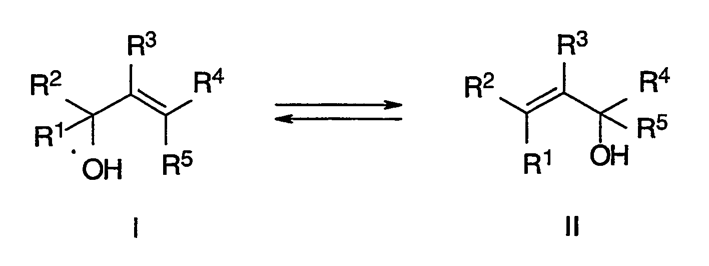 wobei R1 bis R5 Wasserstoff oder einen ein oder mehrfach ungesättigten oder gesättigten C₁-C₁₂-Alkylrest bedeuten, der gegebenenfalls substituiert sein kann,
in beiden Richtungen des Gleichgewichts, dadurch gekennzeichnet, dass die Umsetzung in Gegenwart eines Dioxowolfram(VI)-Komplexes der allgemeinen Formel (III) stattfindet, 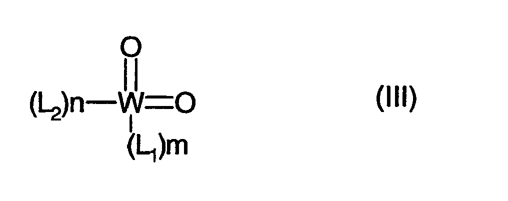 wobei
- L₁, L₂: jeweils unabhängig voneinander einen Liganden ausgewählt aus der Gruppe der Aminoalkohole, der Aminophenole, oder Gemischen davon bedeuten,
- m, n: die Zahl 1 oder 2 bedeutet.

Als mit Hilfe des erfindungsgemäßen Verfahrens vorteilhaft isomerisierbare Allylalkohole der allgemeinen Formel (I) oder (II) seien beispielsweise genannt: 2-Methyl-3-buten-2-ol, Prenol (3-Methyl-2-buten-1-ol), Linalool, Nerol und Geraniol sowie Farnesol (3,7,11-Trimethyl-dodeca-2,6,10-trien-1-ol) und Nerolidol (3,7,11-Trimethyl-dodeca-1,6,10-trien-3-ol), insbesondere Linalool, Nerol und Geraniol.

Die Liganden L1 und/oder L2 können sowohl Liganden wie Aminoalkohole, Aminophenole oder Gemische davon bedeuten.

Als Aminoalkohole können beispielsweise verwendet werden: Triethanolamin, Diethanolamin, Monoethanolamin, Tripropanolamin, Dipropanolamin, 3-Amino-1-propanol, 1-Amino-2-propanol, 2-Amino-1-propanol, Butyldiethanolamin, Methyldiisopropanolamin, N-(2-Hydroxy-benzyl)-amin oder N,N'-Bis-(2-hydroxy-benzyl)-1,2-diaminoethan, α,α.-Diphenyl-L-Prolinol, L-Tryptophanol, L-Alaninol, L-Isoleucinol, L-Leucinol, L-Methioninol, L-Phenylalaninil, L-Valinol, 2-(2-Pyridyl)propan-2-ol

Als Aminophenole können beispielsweise verwendet werden: o-Aminophenol, m-Aminophenol, p-Aminophenol, oder gegebenenfalls durch Halogen, Alkyl, Amino, Hydroxy, Alkoxy, Thio, Sulfonyl, oder Nitro substituiertes 8-Hydroxychinolin, 3-Amino-2-naphtol, N-Methyl-2-aminophenol, N,N-Dimethyl-2-aminophenol, 2-Piperidinophenol, 2-Hydroxypyridin, N-Methyl-2,2'-imino-bis(8-hydroxychinolin), besonders bevorzugt 8-Hydroxychinolin.

Die Phenole können gegebenenfalls durch Halogen, Alkyl, Amino, Hydroxy, Alkoxy, Thio, Sulfonyl oder Nitro substituiert sein.

Vorteilhaft wirkt sich auch die Zugabe von Ammoniak oder auch Aminen zum Edukt Allylalkohol oder auch zur Katalysatorlösung aus.

Unter den Aminen versteht man beispielsweise Mono-, Di- oder Trimethylamin, Ethylamin, Mono-, Di- oder Triethylamin, Mono-, Di-oder Tributylamin, vorzugsweise Diethylamin.

Unter einem Alkohol versteht man, wenn nicht anders angegeben einen Alkohol ROH, wobei R einen ein oder mehrfach ungesättigten oder gesättigten C₁-C₁₅-Alkylrest, der ein oder mehrfach durch Halogen, C₁-C₆-Alkyl, Amino oder Hydroxy substituiert sein kann, beispielsweise Methanol, Ethanol, Propanol, Isopropanol, Butanol, tert.-Butanol, Pentanol, Geraniol, Nerol, Nerolidol, Prenol, Linalool oder Farnesol.

Gegenstand der Erfindung sind auch neue Dioxowolfram(VI)-Komlexe der allgemeinen Formel (III), 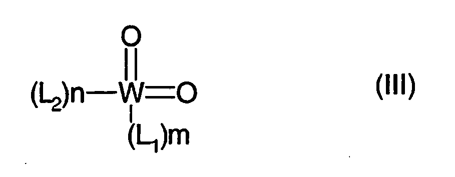 wobei L₁, L₂, n und m die oben angegebene Bedeutung haben mit der Maßgabe, dass für L1, L2 (1R, 2R, 4R) -1,7,7-Trimethyl-2-(2'-pyridinyl)bicyclo[2,2,1]heptan-2-ol, (1S,2S,4S)-1,7,7-Trimethyl-2-(2'pyridinyl)bicyclo-[2,2,1)heptan-2-ol, (1R,2R,4S)-1,3,3-Trimethyl-2-(2'pyridinyl)bicyelo[2.2.1]heptan-2-ol oder (1S,2S,5R)-5-methyl-2-isopropyl-1-(2'-pyridinyl)cyclohexan-1-ol ausgenommen sind.

Insbesonders bevorzugt sind solche Komplexe, wobei die Liganden L1 und/oder L2 8-Hydroxychinolin bedeuten.

Das Molverhältnis Wolfram zu 8-Hydroxychinolin liegt im Bereich von 1 : 1 bis 1 : 5, vorzugsweise 1 : 1 und 1 : 2, insbesonders bevorzugt 1 : 2.

Das Molverhältnis Wolfram zu 8-Hydroxychinolin in der Lösung des Komplexes liegt bei mindestens 1 : 1. Molverhältnisse größer als 1 : 7 sind auch möglich, bringen aber keinen Vorteil mehr.

Die Dioxowolfram(VI)-Komplexe können über die Zwischenstufe des entsprechenden Oxoperoxowolfram(VI)-Komplexes hergestellt werden. Gemäß den in DE 195 33 331 beschriebenen Verfahren wird aus Wolframsäure mit wäßriger Wasserstoffperoxidlösung und gegebenenfalls anschließender Zugabe des oder der Liganden zunächst der Oxoperoxowolfram(VI)-Komplex hergestellt. Dieser wird anschließend zur Wolfram-Oxo-Verbindung reduziert.

Als Reduktionsmittel wird entweder Edukt-Allylalkohol selbst oder ein Reduktionsmittel_beispielsweise Natriumthiosulfat oder Eisen(II)-salze-, insbesondere aber Geraniol und/oder Nerol verwendet.

Der Dioxowolfram(VI)-Komplex kann aber auch ohne die Zugabe einer reduzierend wirkenden Verbindung aus Wolfram(VI)-dioxo-dichlorid hergestellt werden.

Der Dioxowolfram(VI)-Komplex kann sowohl vor der eigentlichen Reaktion separat oder aber auch gleich in situ im Edukt-Allylalkohol hergestellt werden.

Im allgemeinen setzt man den Dioxowolfram(VI)-Komplex im Edukt-Allylalkohol gelöst in einer Konzentration von 0,001 bis etwa 5 Gew.-% ein.

Als besonders vorteilhaft hat es sich erwiesen, den Liganden L1 bzw. L2 mit oder ohne ein zusätzliches organisches Lösungsmittel, zunächst mit der wäßrigen oder organischen Lösung des Dioxowolfram(VI)-Komplexes zu vereinigen und erst dann die derart bereitete Lösung oder Suspension des fertigen Katalysators zum Edukt-Allylalkohol zu geben. Man verwendet den zusätzlichen Liganden im allgemeinen in einer Menge von 1 Mol-% bis 1000 Mol-%, vorzugsweise von 100 Mol-% bis 700 Mol-%, bezogen auf die Wolfram-Menge Größere Mengen Ligand sind zwar auch möglich, bringen aber keinen Vorteil mehr.

Die Absolutkonzentrationen von Ligand und Wolfram-Komplex in der Reaktionsmischung sind im erfindungsgemäßen Verfahren unkritisch und können beispielsweise derart erhöht werden, daß die Geschwindigkeit der Gleichgewichtseinstellung in gewünschter Weise gesteigert wird.

Eine Steigerung der Geschwindigkeit der Gleichgewichtseinstellung kann in dem erfindungsgemäßen Verfahren auch dadurch erzielt werden, daß durch Nebenreaktionen gebildetes Wasser aus der Mischung entfernt wird, beispielsweise durch Überleiten eines Inertgasstromes, Zusatz bekannter Wasser entziehender Mittel oder Strippen mit dem Produkt-Allylalkohol-Gasstrom während der Destillation.

Das erfindungsgemäße Verfahren wird im allgemeinen bei Temperaturen von 50 bis 300°C, vorzugsweise bei 150 bis 250°C, durchgeführt.

Es kann mit und ohne Verwendung eines Lösungsmittels, diskontinuierlich, aber auch kontinuierlich durchgeführt werden. Als Lösungsmittel können organische Lösungsmittel wie Toluol, Tetrahydrofuran, Benzol, Cyclohexan, Xylol, Methylenchlorid oder Mesitylen eingesetzt werden, bevorzugt aber werden die Edukt-Allylalkohole selbst als Lösungsmittel verwendet.

Das erfindungsgemäße Verfahren kann vorteilhaft durchgeführt werden, wenn man die Edukt-Allylalkohole im Reaktionsgemisch in einer Konzentration von etwa 10- Gew.-% bis zu 100 Gew.-% vorliegen hat.

Besonders vorteilhaft gestaltet sich das erfindungsgemäße Verfahren, wenn man als Edukt-Allylalkohole Geraniol und Nerol verwendet und als Produkt-Allylalkohol 2-Linalool herstellt.

Zur Aufarbeitung wird hierbei 2-Linalool als niedriger siedende Komponente durch Destillation vom Produktgemisch abgetrennt. Im allgemeinen werden Edukt-Allylalkohole und Nebenverbindungen im Produkt-Allylalkohol enthalten sein. Eine Reindestillation des Produkt-Allylalkohols kann nach bekannten Verfahren erfolgen.

Die Isomerisierung stellt eine Gleichgewichtsreaktion dar und die Gleichgewichtslage hängt von den thermodynamischen Eigenschaften der Edukt- und Produkt-Allylalkohole sowie von den Reaktionsbedingungen ab. Eine diskontinuierliche oder kontinuierliche Entfernung von Linalool, dem im Gemisch am niedrigsten siedenden Allylalkohol aus dem Reaktionsansatz erlaubt auch bei ungünstiger Gleichgewichtseinstellung aufgrund der Verschiebung des Gleichgewichtes eine günstige Raum-Zeit-Ausbeute, wobei der Sumpf der Destillationskolonne als Reaktionsraum dient.

Die folgenden Beispiele sollen das erfindungsgemäße Verfahren näher beschreiben ohne es jedoch darauf einzuschränken:
Herstellung eines cis-Dioxowolfram(VI)-Komplexes am Beispiel des cis-Dioxowolfram(VI)-bis(oxinat)

### Beispiel 1

Beispielsweise wird Wolframsäure bei 40 °C in einem 3,5-fachen Überschuss wäßrigem Wasserstoffperoxid suspendiert. Nach 6-stündigem Rühren wird die Lösung filtriert. Zu dieser bereiteten Oxoperoxowolfram(VI)-Lösung wird 8-Hydroxychinolin gegeben. Das 8-Hydroxychinolin wird als Feststoff oder als Schmelze zugesetzt oder gelöst in einem organischen Lösungsmittel, z.B. Alkohol, zugetropft. 8-Hydroxychinolin wird in einer bevorzugten Menge von 1 bis 3,5 Mol-Äquivalenten in Bezug auf das Wolfram eingesetzt. Die erhaltene Lösung oder Suspension wird direkt für die Isomerisierung mit einem Edukt-Allylalkohol eingesetzt, indem diese zu Geraniol und/oder Nerol gegeben wird. Die Oxoperoxowolfram(VI)-Komplex-Verbindung reagiert mit dem Edukt-Allylalkohol zur cis-Dioxowolfram(VI)-Komplex-Verbindung und Geraniol- und/oder Nerolepoxid. Die erhaltene Mischung kann direkt für die weitere Isomerisierung eingesetzt werden oder aber auch durch Abkühlen oder Einengen der cis-Dioxowolfram (VI)-Komplex-Verbindung mit 8-Hydroxychinolin als Liganden gefällt werden. Das gefällte Katalysatorpulver wird abfiltriert und nach dem Waschen getrocknet. Gegebenenfalls in der Katalysatorlösung vorhandenes Wasser kann durch Destillation oder durch Zugabe von Trocknungsmittel entfernt werden. Der nach oben beschriebenen Verfahren vorbereitete neue Wolfram-Komplex enthält zwei Oxo-Gruppen und 8-Hydroxychinolin als weiteren Liganden und ist dadurch gekennzeichnet, dass Hydroxychinolin an der Phenol-OH-Gruppe deprotoniert vorliegt und dass das Verhältnis Wolfram zu 8-Hydroxychinolin 1:2 beträgt.

Dieser Katalysator kann bei Temperaturen von 20 bis 300°C gelöst in einem beliebigen Lösungsmittel oder als Feststoff zum Edukt-Allylalkohol gegeben werden.

### Beispiel 2

Cis-Dioxowolfram(VI)-bis(oxinat) kann aber auch ohne die Zugabe einer reduzierend wirkenden Verbindung hergestellt werden

Hierzu werden in einem 50 ml Autoklaven unter 2 bar Ammoniak-Gas 2,0 g Wolfram(VI)-dioxo-dichlorid mit 10 g Methanol und 2,1 g 8-Hydroxychinolin vermischt und 24 Stunden auf 90°C hochgeheizt. Nach dem Abkühlen wird der Feststoff abgesaugt und mit Methanol gewaschen. Der Filterkuchen wird zweimal in je 50 g 50 Gew.-%igem wäßrigen Methanol aufgekocht und nach dem Abkühlen erneut filtriert und mit Methanol gewaschen. Das zurückbleibende Pulver aus Cis-Dioxowolfram(VI)-bis-(oxinat) wird im Luftstrom des Wasserstrahlvakuums getrocknet.

Die Ausbeute beträgt 89 %.

| | |
|---|---|
| Mikroanalyse: | C: 42,3 %; O: 12,7 %; N: 5,4 %; H: 1,9 %; W: 38 %; Cl org.: 0,056 %; C1 anorg.: 0,038 % |

### Beispiel 3

Das nach Beispiel 1 hergestellte Katalysatorpulver wird direkt für die Isomerisierung von Geraniol eingesetzt. Hierzu werden unter Argon 50 g Geraniol in einem 100 ml Dreihalskolben mit aufgesetzter Destillationsbrücke auf 180°C erhitzt. Anschließend werden 0,14 g des getrockneten Katalysatorpulvers zugegeben und 1 Stunde bei 180°C gerührt. Während der Isomerisierung wird kein Produkt abdestilliert. Nach dem Abkühlen wird vom Sumpf eine GC-Analyse durchgeführt.

| | |
|---|---|
| GC-Analyse in GC-F1.-%: | Geraniol: 57,35 %; Nerol: 4,45 %; Linalool: 35,20 %; Leichtsieder: 0,55 %; Citral: 0,18 %; Hochsieder: 2,37 % |

## Patentansprüche

1. Verfahren zur Isomerisierung von Edukt-Allylalkoholen der Formel (I) zu Produkt-Allylalkoholen der allgemeinen Formel (II) 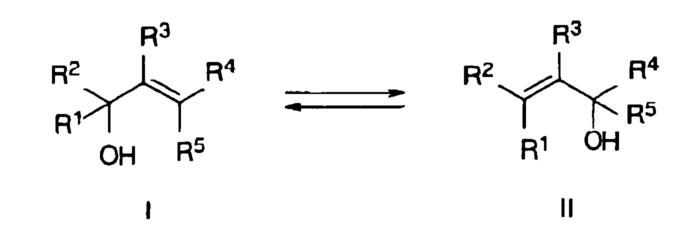 wobei R1 bis R5 Wasserstoff oder einen ein oder mehrfach ungesättigten oder gesättigten C₁-C₁₂-Alkylrest bedeuten, der gegebenenfalls substituiert sein kann,
in beiden Richtungen des Gleichgewichts, **dadurch gekennzeichnet, dass** die Umsetzung in Gegenwart eines Dioxowolfram(VI)-Komplexes der allgemeinen Formel (III) stattfindet, 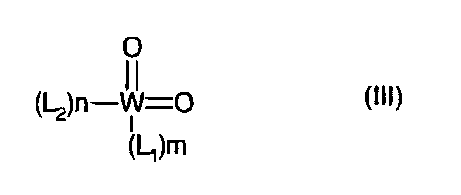 wobei
L₁, L₂ jeweils unabhängig voneinander einen Liganden ausgewählt aus der Gruppe der Aminoalkohole, der Aminophenole oder Gemischen davon bedeutet
m, n die Zahl 1 oder 2 bedeutet.

2. Verfahren gemäß einem der Ansprüche 1 oder 2, **dadurch gekennzeichnet, dass** L1 und L2 8-Hydroxychinolin bedeuten.

3. Verfahren gemäß einem der Ansprüche 1 oder 2, **dadurch gekennzeichnet, dass** das Verhältnis Wolfram zu 8-Hydroxychinolin im Komplex zwischen 1 : 1 und 1 : 5 liegt.

4. Verfahren gemäß einem der Ansprüche 1 bis 3, **dadurch gekennzeichnet, dass** das Verhältnis Wolfram zu 8-Hydroxychinolin in der Lösung des Komplexes bei mindestens 1 : 1 liegt.

5. Verfahren gemäß einem der Ansprüche 1 bis 4, **dadurch gekennzeichnet, dass** die Allylalkohole ausgewählt sind aus der Gruppe 2-Methyl-3-buten-2-ol, Prenol (3-Methyl-2-buten-1-ol), Linalool, Nerol, Geraniol, Farnesol (3,7,11-Trimethyl-do-deca-2,6,10-trien-1-ol) oder Nerolidol (3,7,11-Trimethyl-dodeca-1,6,10-trien-3-ol).

6. Verfahren gemäß einem der Ansprüche 1 bis 5, **dadurch gekennzeichnet, dass** die Edukt-Allylalkohole ausgewählt sind aus der Gruppe Geraniol oder Nerol.

7. Verfahren gemäß einem der Ansprüche 1 bis 6, **dadurch gekennzeichnet, dass** der Dioxowolfram(VI)-Komplex der allgemeinen Formel (III) vor der Reaktion oder in situ im Edukt-Allylalkohol hergestellt wird.

8. Verfahren gemäß einem der Ansprüche 1 bis 7, **dadurch gekennzeichnet, daß** zum Edukt-Allylalkohol, zum Katalysator oder Zu einer Mischung aus Edukt-Allylalkohol und Katalysator Ammoniak oder Amin gegeben wird.

9. Dioxowvlfram(VI)-Komplexe der allgemeinen Formel (III), 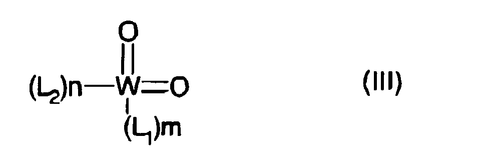 wobei
L1, L2 jeweils unabhängig voneinander einen Liganden ausgewählt aus der Gruppe der Aminoalkohole, der Aminophenole oder Gemischen davon bedeutet,
m, n die Zahl 1 oder 2 bedeutet,
mit der Maßgabe, dass für L1, L2 (1R,2R,4R)-1,7,7-Trimethyl-2-(2'-pyridinyl) bicyclo[2,2,1] heptan-2-ol, (1S,2S,4S)-1,7,7-Trimethyl-2-(2'pyridinyl)bicyclo-[2,2,1]heptan-2-ol, (1R,2R,4S)-1,3,3-Trimethyl-2-(2'pyridinyl)bicyclo[2.2.1]heptan-2-ol oder (1S,2S,5R)-5-methyl-2-isopropyl-1-(2'-pyridinyl)cyclohexan-1-ol ausgenommen sind.

10. Dioxowolfram(VI)-Komplexe gemäß Anspruch 9, **dadurch gekennzeichnet, dass** L1 und/oder L2 8-Hydroxychinolin bedeuten.

11. Verwendung der Verbindungen der allgemeinen Formel (III) gemäß Anspruch 1 als Katalysatoren bei der Isomerisierung von Allylalkohole.

## Claims

1. A process for the isomerization of precursor allyl alcohols of the formula (I) to product allyl alcohols of the formula (II) 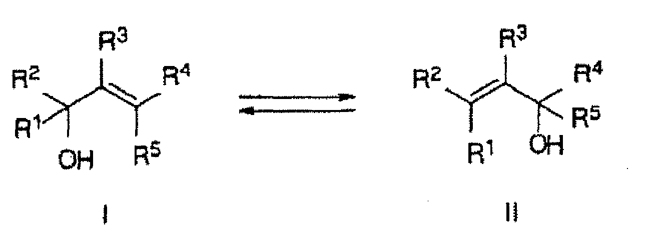 where R¹ and R⁵ are hydrogen or a mono- or polyunsaturated or saturated C₁-C₁₂-alkyl radical, which may be optionally substituted,
in both direction of the equilibrium, wherein the reaction takes place in the presence of a dioxotungsten(VI) complex of the formula (III), 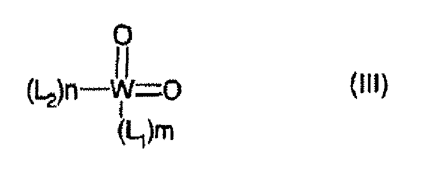 where
L1, L2 in each case independently of one another are a ligand chosen from the group of amino alcohols, aminophenols or mixtures thereof
m, n are the number 1 or 2.

2. The process according to any of claims 1 or 2, wherein L1 and L2 are 8-hydroxyquinoline.

3. The process according to any of claims 1 or 2, wherein the ratio of tungsten to 8-hydroxyquinoline in the complex is between 1:1 and 1:5.

4. The process according to any of claims 1 to 3, wherein the ratio of tungsten to 8-hydroxyquinoline in the solution of the complex is at least 1:1.

5. The process according to any of claims 1 to 4, wherein the allyl alcohols are chosen from the group
2-methyl-3-buten-2-ol, prenol (3-methyl-2-bunten-1-ol), linalool, nerol, geraniol, farnesol
(3-7,11-trimethyldodeca-2,6,10-triene-1-ol) and nerolidol
(3-7,11-trimethyldodeca-1,6,10-trien-3-ol) and nerolidol
(3,7,11-trimethyldodeca-1,6,10-triene-3-ol) .

6. The process according to any of claims 1 to 5, wherein the precursor allyl alcohols are chosen from the group geraniol and nerol.

7. The process according to any of claims 1 to 6, wherein the dioxotungsten(VI) complex of the formula (III) is prepared prior to the reaction or in situ in the precursor allyl alcohol.

8. The process according to any of claims 1 to 7, wherein ammonia or amine is added to the precursor allyl alcohol, to the catalyst or to a mixture of precursor allyl alcohol and catalyst.

9. A dioxotungsten(VI) complex of the formula (III), 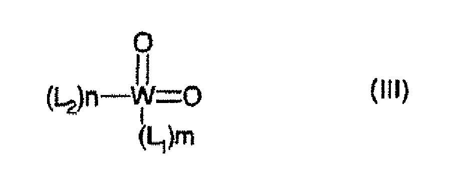 where
L1, L2 in each case independently of one another are a ligand chosen from the group of amino alcohols, aminophenols or mixtures thereof,
m, n are the number 1 or 2,
with the proviso that
(1R,2R,4R)-1,7,7-trimethyl-2-(2'-pyridinyl)bicyclo[2,2,1]heptan-2-ol,
(1S, 2S, 4S)-1,7,7-trimethyl-2-(2'-pyridinyl)bicyclo[2,2,1]heptan-2-ol,
(1R,2R,4S)-1,3,3-trimethyl-2-(2'-pyridinyl)bicyclo[2.2.1]heptan-2-ol or
(1S,2S,5R)-5-methyl-2-isopropyl-1-(2'-pyridinyl)cyclohexan-1-ol are excluded for L1, L2.

10. The dioxotungsten(VI) complex according to claim 9, wherein L1 and/or L2 are 8-hydroxyquinoline.

11. The use of compounds of the formula (III) according to claim 1 as catalysts in the isomerization of allyl alcohols.

## Revendications

1. Procédé d'isomérisation d'alcools allyliques précurseurs de formule (I) en produits alcools allyliques de formule générale (II) 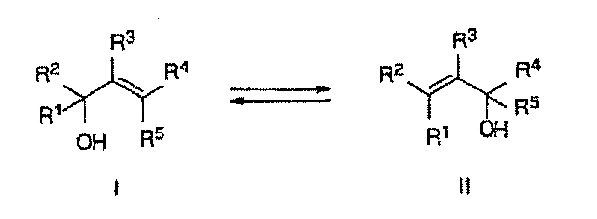 dans laquelle R1 à R5 désignent l'hydrogène ou un reste alkyle en C₁ à C₁₂ mono- ou polyinsaturé ou saturé qui peut éventuellement être substitué,
dans les deux directions de l'équilibre, **caractérisé en ce que** la mise en réaction se déroule en présence d'un complexe de dioxotungstène (VI) de formule générale (III) 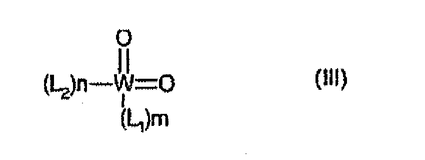 dans laquelle :
L1 L2 désignent respectivement, indépendamment l'un de l'autre, un ligand choisi dans le groupe des aminoalcools, des aminophénols ou des mélanges de ceux-ci,
m, n désignent le nombre 1 ou 2.

2. Procédé selon l'une des revendications 1 ou 2, **caractérisé en ce que** L1 et L2 désignent la 8-hydroxyquinoléine.

3. Procédé selon l'une des revendications 1 ou 2, **caractérisé en ce que** le rapport du tungstène à la 8-hydroxyquinoléine dans le complexe se situe entre 1:1 et 1:5.

4. Procédé selon l'une des revendications 1 à 3, **caractérisé en ce que** le rapport du tungstène à la 8-hydroxyquinoléine dans la solution du complexe est au moins 1:1.

5. Procédé selon l'une des revendications 1 à 4, **caractérisé en ce que** les alcools allyliques sont choisis dans le groupe 2-méthyl-3-butén-2-ol, le prénol (3-méthyl-2-butén-1-ol), le linalool, le nérol, le géraniol, le farnésol (3,7,11-triméthyl-dodéca-2,6,10-trién-1-ol) ou le nérolidol (3,7,11-triméthyl-dodéca-1,6,10-trién-3-ol).

6. Procédé selon l'une des revendications 1 à 5, **caractérisé en ce que** les alcools allyliques précurseurs sont choisis dans le groupe géraniol ou nérol.

7. Procédé selon l'une des revendications 1 à 6, **caractérisé en ce que** le complexe de dioxotungstène (VI) de formule générale (III) est préparé avant la réaction ou in situ dans l'alcool allylique précurseur.

8. Procédé selon l'une des revendications 1 à 7, **caractérisé en ce que** de l'ammoniac ou une amine est ajouté à l'alcool allylique précurseur, au catalyseur ou à un mélange d'alcool allylique précurseur et de catalyseur.

9. Complexes de dioxotungstène (VI) de formule générale (III) 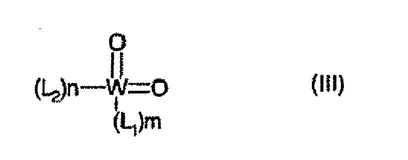 dans laquelle :
L1, L2 désignent respectivement, indépendamment l'un de l'autre, un ligand choisi dans le groupe des aminoalcools, des aminophénols ou des mélanges de ceux-ci,
m, n désignent le nombre 1 ou 2,
à condition que, pour L1 L2, soient exclus :
le (1R,2R,4R)-1,7,7-triméthyl-2-(2'-pyridinyl)bicyclo[2,2,1]heptan-2-ol,
le (1S,2S,4S)-1,7,7-triméthyl-2-(2'-pyridinyl)bicyclo[2,2,1]heptan-2-ol,
le (1R,2R,4S)-1,3,3-triméthyl-2-(2'-pyridinyl)bicyclo[2,2,1]heptan-2-ol, ou
le (1S,2S,5R)-5-méthyl-2-isopropyl-1-(2'-pyridinyl)-cyclohexan-1-ol.

10. Complexes de dioxotungstène (VI) selon la revendication 9, **caractérisés en ce que** L1 et/ou L2 désignent la 8-hydroxyquinoléine.

11. Utilisation des composés de formule générale (III) selon la revendication 1 comme catalyseurs pour l'isomérisation d'alcools allyliques.
